# EUROPEAN PATENT APPLICATION

(11) **EP 0 848 008 A1**
(43) Date of publication of application: **17.06.1998**
(21) Application number: 96203495.5
(22) Date of filing: 10.12.1996
(51) Int. Cl.: C07F 7/22, A61K 31/32

(54) **Organotin compounds and compositions containing these compounds**

(71) Applicant: PHARMACHEMIE B.V., 2031 GA Haarlem (NL)
(72) Inventor: Gielen, Marcel, 1970 Wezenbeek-Oppem (BE); Dalil, Hassan, 1080 Brussel (BE); Willem, Rudolph, 1800 Vilvoorde (BE); Biesemans, Monique, 2890 St. Amands (BE); De Vos, Dick, 2341 LP Oegstgeest (NL)
(74) Representative: Ellowicz, Leo, Drs.

(57) **Abstract**

This invention provides novel organotin compounds, which are derivatives of terebic acid, (H₉O₂O₂C₆-COOH), which compounds exhibit excellent activities against various tumours. This invention also provides anti-tumour compositions which contain said novel organotin compounds in combination with a suitable carrier.

## Description

The present invention relates to novel organotin compounds described by the formula I 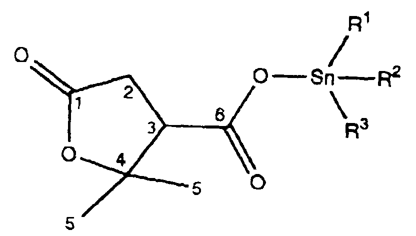 wherein R¹, R² and R³ may be the same or different groups and represent C₁-C₄ alkyl or C₆H₅, and in addition one of R¹, R² and R³ may represent a group described by the formula II 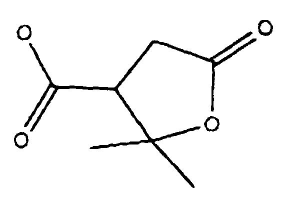 which compounds show a strong anti-tumour activity as will be illustrated hereinafter.

The present invention also provides anti-tumour compositions, containing a compound as defined above in combination with a suitable carrier.

Di-n-butyltin diterebate, (H₉O₂C₆-COO)₂Sn(CH₂CH₂CH₂CH₃)₂, compound **1**, tri-n-butyltin terebate, (H₉O₂C₆-COOSn(CH₂CH₂CH₂CH₃)₃, compound **2**, and triphenyltin terebate, (H₉O₂C₆-COO(C₆H₅)₃, compound **3**, were synthesized by the condensation of dl-terebic acid, (H₉O₂C₆-COOH), with di-n-butyltin oxide, tri-n-butyltin acetate and triphenyltin hydroxide, respectively.
Compound 1: Formula I wherein R¹=R²-CH₂CH₂CH₂CH₃ and R³ is 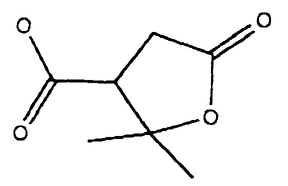
Compound 2: Formula I, R¹=R²=R³=CH₂CH₂CH₂CH₃
Compound 3: Formula I, R¹=R²=R³=C₆H₅
These compounds were prepared by adding 5 mmole of di-n-butyltin oxide, tri-n-butyltin acetate or triphenyltin hydroxide, respectively, to a solution of 10 mmole, 5 mmole or 5 mmole of terebic acid in 150 cm³ toluene and 50 cm³ ethanol. After refluxing for 6 hours and distilling off the ternary azeotrope with a Dean Stark funnel and half of the remaining solvent, the resulting mixture was cooled to room temperature, filtered and evaporated under vacuum. The residue was recrystallized from a suitable solvent.

### Compound 1

Recrystallized from chloroform/n-hexane; mp: 98-100°C; yield: 85%
Mössbauer parameters (in mm/s): QS: 3.44; IS: 1.34; Γ₁: 0.99; Γ₂: 0.91
¹H NMR (CDCl₃): 2.988, dd (18, 9): H-2a; 2.700, dd (18, 9): H-2b; 3.196, dd (9, 9): H-3; 1.581, s: H-5a; 1.348, s: H-5b; 1.62 - 1.64, m: H-α & H-β; 1.25 - 1.40, m: H-γ; 0.873, t (7): H-δ; ¹³C NMR (CDCl₃): C-1: 174.1; C-2: 32.5; C-3: 50.5; C-4: 84.5; C-5a: 28.5; C-5b: 23.5; C-6: 179.5; C-α: 25.9 [¹J(^{119/117}Sn-¹³C) = 573/550]; C-β: 26.7 [²J(^{119/117}Sn-¹³C) ≈ 31]; C-γ: 26.4 [³J(^{119/117}Sn-¹³C) = 100/96]; C-δ: 13.5
¹¹⁹Sn NMR (CDCl₃): -131.6.

### Compound 2

Recrystallized from petroleum ether; mp: 50-51°C; yield: 95%
Mössbauer parameters (in mm/s): QS: 3.77; IS: 1.47; Γ₁: 0.85; Γ₂: 0.86
¹H NMR (CDCl₃): 2.985, dd (17, 10): H-2a; 2.622, dd (17, 8): H-2b; 3.121, dd (10, 8): H-3; 1.562, s: H-5a; 1.297, s: H-5b; 1.50 - 1.65, m: H-α & H-β; 1.20 - 1.40, m: H-γ; 0.878, t (7): H-δ
¹³C NMR (CDCl₃): C-1: 174.4; C-2: 32.7; C-3: 51.4; C-4: 84.8; C-5a: 28.5; C-5b: 23.3; C-6: 174.8; C-α: 16.7 [¹J(^{119/117}Sn-¹³C) = 356/338]; C-β: 27.8 [²J(^{119/117}Sn-¹³C) ≈ 21]; C-γ: 27.0 [³J(^{119/117}Sn-¹³C) ≈ 64]; C-δ: 13.6
¹¹⁹Sn NMR (CDCl₃): 125.4.

### Compound 3

Recrystallized from ethanol/petroleum ether; mp: 125-126°C; yield: 95%;
Mössbauer parameters (in mm/s): QS: 3.52; IS: 1.31; Γ₁: 0.82; Γ₂: 0.79
¹H NMR (CDCl₃): 3.065, dd (18, 10): H-2a; 2.669, dd (18, 9): H-2b; 3.231, dd (10, 9): H-3; 1.585, s: H-5a; 1.114, s: H-5b; 7.70-7.80, m: H-*o*; 7.30-7.50, m: H-*m* & H-*p*
¹³C NMR (CDCl₃) : C-1: 174.5; C-2: 32.7; C-3: 50.8; C-4: 84.8; C-5a: 28.5; C-5b: 23.2; C-6: 175.6; C-*i*: 137.5 [¹J(^{119/117}Sn-¹³C) = 648/618]; C-*o*: 136.9 [²J(^{119/117}Sn-¹³C) = 51/49]; C-*m*: 129.1 [³J(^{119/117}Sn-¹³C) = 65/62]; C-*p*: 130.5 [⁴J(^{119/117}Sn-¹³C) ≈ 13]
¹¹⁹Sn NMR (CDCl₃): -97.6.

Compounds 1, 2 and 3 were tested in vitro. together with 2 reference compounds used clinically (carboplatin and cisplatin), against MCF-7 and EVSA-T, two breast cancers, WiDr, a colon cancer, IGROV, an ovarian cancer, M19MEL, a melanoma, A498, a renal cancer, and H226, a non small cell lung cancer.
The tests were carried out according to the method of Skehan et al, Journal of the National Cancer Institute, 1990; 82: 1107-1112.
The anti-tumour activities are summarized in the following table, which shows the ID₅₀ values, expressed in ng/ml (the amounts which inhibit 50% of the cell growth).

From the results it can be seen that the present compounds have excellent antitumour activities which are considerably better than those of the known compounds which were tested for comparative purposes.

| Compounds | MCF-7 | EVSA-T | WiDr | IGROV | M19MEL | A498 | H226 |
|---|---|---|---|---|---|---|---|
| **1** | 27 | 25 | 134 | 18 | 61 | 61 | 104 |
| **2** | 3 | <3 | 11 | 4 | 11 | 15 | 8 |
| **3** | 17 | <3 | 17 | 19 | 42 | 42 | 39 |
| Carboplatin | 10500 | 4500 | 3500 | 2400 | 5500 | 18000 | 25000 |
| Cisplatin | 1400 | 920 | 1550 | 230 | 780 | 1200 | 3158 |

## Claims

1. Organotin compounds described by the formula I 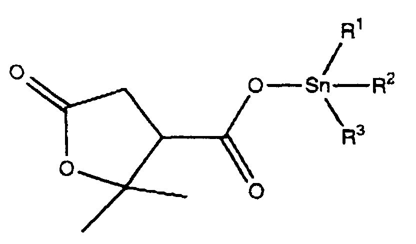 wherein R¹, R² and R³ may be the same or different groups and represent
C₁-C₄ alkyl or C₆H₅, and in addition one of R¹, R² and R³ may represent a group described by the formula II

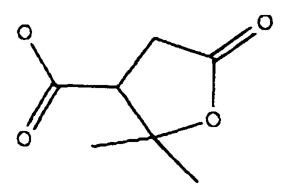

2. Anti-tumour compositions, containing a compound as defined in claim 1 as an active ingredient and a suitable carrier, optionally in combination with further additives.
